# EUROPÄISCHE PATENTSCHRIFT

(11) **EP 0 711 568 B1**
(45) Veröffentlichungstag und Bekanntmachung des Hinweises auf die Patenterteilung: **30.09.1998**
(21) Anmeldenummer: 96101316.6
(22) Anmeldetag: 19.03.1993
(51) Int. Cl.: A61M 1/00, A61M 25/00, A61M 16/04

(54) **Absaugkatheter**
Suction catheter
Cathéter d'aspiration

(30) Priorität: 20.03.1992 DE 4208912
(43) Veröffentlichungstag der Anmeldung: 15.05.1996
(62) Teilanmeldung aus: 93906567.8
(73) Patentinhaber: Schön, Rudolf, D-53127 Bonn (DE)
(72) Erfinder: Schön, Rudolf, D-53127 Bonn (DE)
(74) Vertreter: Müller-Gerbes, Margot, Dipl.-Ing.

(56) Entgegenhaltungen:
- US-A- 2 574 135
- US-A- 3 848 604

## Beschreibung

Die Erfindung betrifft einen Absaugkatheter zum Absaugen von Schleim und anderen Fluiden aus dem Tracheobronchialraum eines Patienten, enthaltend ein biegsames Katheterrohr mit einem proximalen Ende und einem distalen Ende und mindestens einem durchgehend ausgebildeten Lumen, wobei das Katheterrohr in einem distalen Endbereich Belüftungsöffnungen aufweist und das Lumen des Katheterrohres am distalen Ende eine trichterartige Erweiterung bildet.

Absaugkatheter der gattungsgemäßen Art sind beispielsweise aus der DE-B-2364119 bekannt.

Aus der US-A-2130 406 ist ein Saugschnorchel zum Absaugen von Speichel aus dem Mundraum für zahnärztliche Zwecke bekannt, der an seinem distalen Ende außenseitig verdickt ist, wobei die Verdickung durch radial verlaufende Kerben außenseitig unterbrochen ist.

Einen ähnlich gestalteten Saugschnorchel für zahnärztliche Zwecke zeigt die US-A-2574 135. Hierbei ist das Schnorchelende in Gestalt eines zylindrischen Kolbens verdickt und weist außenseitige, bis an die distale Austrittsöffnung reichende Kerben auf.

Beim Absaugen von Schleim aus dem Tracheobronchialraum mittels Absaugkathetern kann ein andauerndes Vakuum während des Absaugens zu ernsten Verletzungen der Schleimhaut führen. Zum einen besteht die Gefahr, daß der Absaugkatheter an der Schleimhaut klebt und direkt von ihr fortgezogen wird. Absaugkatheter sind üblicherweise zusätzlich zu der distalen Austrittsöffnung am Ende mit mindestens einem Seitenloch ausgestattet. Wenn der Absaugkatheter in enge Berührung mit der Schleimhaut kommt, hebt er durch den Unterdruck die Schleimheit an und invaginiert sie in diese Seitenlöcher oder auch in die Austrittsöffnung. Die dadurch entstehenden Verletzungen dienen als Brutstätte für Bakterien und führen zu Ödemen usw.

Bei dem aus der DE-B-2364119 bekannten Katheter ist zur Vermeidung von Verletzungen der Schleimhaut am distalen Ende des Absaugkatheters ein abgerundeter Ringwulst ausgebildet, der radial am Ende des Katheters über den Umfang des Katheters vorsteht, wobei das Lumen des Katheterrohres am distalen Ende etwas trichterartig im Übergang zu dem Ringwulst ausgebildet ist. Mit Hilfe dieses Ringwulstes wird der Katheter innerhalb der Luftröhre zentriert und soll des weiteren ein laminares Luftpolster schaffen, damit er nicht mit der Wand der Luftröhre in Berührung kommt und auch keine Schleimhaut in den Katheter eingesogen werden soll. Es hat sich jedoch herausgestellt, daß nicht in allen Fällen das Einziehen von Schleimhaut in die radialen seitlichen Öffnungen des Katheters vermieden werden kann. Darüber hinaus stellt sich in manchen Fällen das Problem, daß die distale Austrittsöffnung durch Schleim verstopft wird.

Der Erfindung liegt die Aufgabe zugrunde, die bekannten Absaugkatheter konstruktiv zu verbessern, so daß ein Verstopfen des distalen Endes durch Schleim beim Absaugen vermieden wird und auch das Festkleben oder Einsaugen von Schleimhaut in die distale Austrittsöffnung bzw. die seitlichen Öffnungen im Bereich des distalen Katheterendes mit Sicheheit vermieden werden.

Zur Lösung dieser Aufgabe wird vorgeschlagen, den gattungsgemäßen Absaugkatheter in der Weise weiterzubilden, daß in der die trichterartige Erweiterung begrenzenden Wandung des Katheterrohres mindestens eine sich axial erstreckende Kerbe vorhanden ist.

So ist es möglich, die trichterartige Erweiterung und gegebenenfalls die Lumenwandungen mittels in die Wandungen eingedrückter in axialer Richtung verlaufende Kerben zu erweitern. Die Kerben in der trichterförmigen Erweiterung können sich gleich weit, weniger weit oder weiter als die trichterförmige Erweiterung in axialer Richtung in das Lumen hinein erstrecken.

Überraschend hat sich herausgestellt, daß auch bei sehr zähem Sekret, das mit Hilfe des Absaugkatheters aus dem Bronchialraum abgesaugt werden soll, bei Ansetzen des Unterdruckes das Sekret die distale Austrittsöffnung des Absaugkatheters nicht verstopft, sondern zügig abgesaugt wird. Zugleich wird auch das Festsaugen des Absaugkatheters bzw. des distalen Endbereiches an den Schleimhäuten vermieden. Jede, auch die geringste Invagination der Schleimhäute wird vermieden. Dies wird darauf zurückgeführt daß infolge der in der trichterförmigen Erweiterung des Lumens vorhandenen Kerben zur distalen Austrittsöffnung hin ein verbessertes Ansaugen des Schleimes und Ableitung in das Lumen erfolgt. Die distale Austrittsöffnung des Absaugkatheters ist erfindungsgemäß mittels mindestens einer Kerbe in einem Teilbereich erweitert, die in die Wandungen der trichterförmigen Erweiterung eingedrückt ist. Diese Kerbe oder Kerben sind relativ klein ausgeführt und können über den erweiterten Austrittsbereich bis in den normalen Lumenbereich hin ausgebildet sein. Bei einer bevorzugten Ausgestaltung reichen die Kerben gleich weit, vom distalen Ende des Katheterrohres her betrachtet, wie die gleichfalls eingeformte trichterförmige Erweiterung in das Lumen in axialer Richtung hinein.

Für die einwandfreie Funktion des erfindungsgemäßen Absaugkatheters als atraumatischer Katheter ist die Größe der trichterförmigen Erweiterung andererseits ausschlaggebend.

Die Erweiterung des Lumens kann kegelförmig oder trompetenförmig aufgeweitet sein und ist mittels in die Lumenwandungen eingedrückter bis zum distalen Ende des Katheterrohres reichender Kerben ausgebildet. Einige erfinderische Ausgestaltungen eines Absaugkatheters der gattungsgemäßen Art mit erweiterter distaler Austrittsöffnung sind in den Ansprüchen 2 bis 4 gekennzeichnet.

Der Absaugkatheter bzw. das Katheterrohr sind aus einem nichttoxischen flexiblen biegsamen Material, wie beispielsweise natürlichem oder synthetischem Gummi, Polypropylen, Polyethylen, Polyvinylchlorid oder Polyamiden, hergestellt.

Hierbei kann die Steifigkeit des Materials so gewählt werden, daß auch bei langen Absaugkathetern, die in der Regel bei den angegebenen Größen von 8 CH bis 18 CH eine Länge zwischen 50 bis 65 cm aufweisen, eine ausreichende Steifigkeit und Knicksicherheit erreicht wird, auch bei sehr geringen Wandstärken des Katheterrohres.

Das proximale Ende des Absaugkatheters kann mit einem Anschlußstück, beispielsweise Trichter, versehen sein, jedoch auch mit anderen Teilen ausgerüstet oder an Geräte angeschlossen werden. Das proximale Ende des Absaugkatheters kann selbst auch konisch erweitert ausgebildet werden.

Der distale Endbereich des Absaugkatheters ist üblicherweise in gerade Längserstreckung des Katheterrohres vorgesehen, er kann jedoch auch in bezug auf die Längsachse des Absaugkatheters ein- oder mehrfach abgebogen ausgebildet sein.

Die Erfindung wird in der Zeichnung anhand eines Ausführungsbeispieles näher erläutert. Es zeigen
- Fig. 1: in schematischer Darstellung im Längsschnitt nicht maßstabgerecht einen Absaugkatheter, bei dem der Übersichtlichkeit wegen die erfindungsgemäße Kerbe nicht dargestellt ist,
- Fig. 2: eine Aufsicht auf eine weitere Ausgestaltung des distalen Endbereiches des Absaugkatheters
- Fig. 3: den Schnitt AA nach Fig. 2
- Fig. 4: den Schnitt BB nach Fig. 2 für den distalen Endbereich.

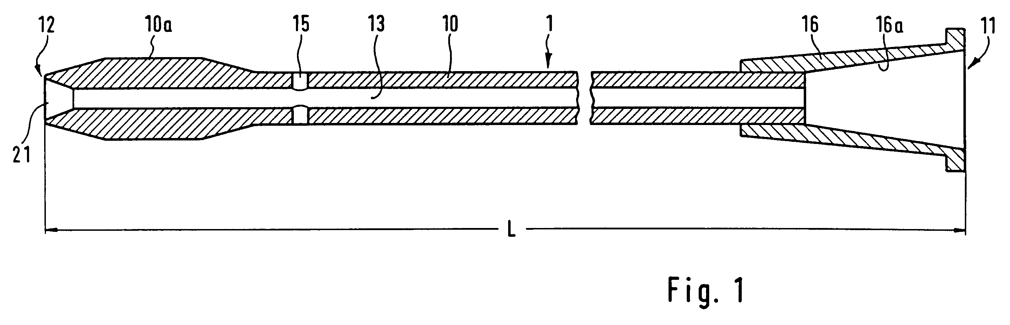

In der Fig. 1 ist schematisch ein Absaugkatheter 1 zum Absaugen von Sekreten und anderen Fluiden aus dem Tracheobronchialraum eines Patienten dargestellt, der durch die Luftröhre des Patienten bis in den Bronchialraum einführbar ist. Der Absaugkatheter ist an seinem proximalen Ende 11 in dem gezeigten Beispiel mit einem Anschlußstück 16, beispielsweise Trichter mit Innentrichter 16a fest verbunden. Der Absaugkatheter kann auch mit anderen Anschlußstücken verbunden werden. Der Absaugkatheter 1 enthält das Katheterrohr 10, das innerhalb des distalen Endbereiches mit einer verdickung in Gestalt eines zylindrischen Kolbens 10a ausgebildet ist. Das einzige durchgängige in dem Katheterrohr ausgebildete Lumen 13 des Absaugkatheters 1 mündet am distalen Ende 12 in der distalen Austrittsöffnung 21. Absaugkatheter der dargestellten Art weisen eine Länge L üblicherweise zwischen etwa 50 bis 65 cm auf. Der Innendurchmesser des durchgängigen Lumens 13 beträgt beispielsweise bei einer Kathetergröße CH 10 1,54 mm und bei einer Kathetergröße CH 16 3,0 mm.

An den Kolben 10a des Absaugkatheters schließen sich in Richtung auf das proximale Ende 11 des Absaugkatheters relativ nahe Belüftungsöffnungen 15 an, die sich radial durch die Wandung des Katheterrohres 10 erstrecken und die das Lumen 13 mit der Umgebung verbinden. Hierbei sind die Belüftungsöffnungen 15 als Bohrungen gleichmäßig über den Umfang des Katheterrohres 10 verteilt angeordnet, beispielsweise zwei oder vier Belüftungsöffnungen 15.

Das durchgängige Lumen 13, das in der Längsache des Absaugkatheters 1 koaxial verläuft, ist zum distalen Ende 12 hin trichterförmig erweitert. Diese trichterförmige Erweiterung 21a mündet in der Austrittsöffnung 21. Die Austrittsöffnung 21 des durchgängigen Lumens 13 am distalen Ende 12 des Absaugkatheters 1 ist damit größer im Querschnitt als der Querschnitt des Lumens 13. Die trichterförmige Erweiterung des Lumens des Katheterrohres ist von geraden oder leicht gerundeten Wänden begrenzt.

Wenn nun der Absaugkatheter 1 in ein Bronchialsystem zum Absaugen eingeführt ist und Vakuum angelegt wird, wird Sekret in Richtung auf die distale Austrittsöffnung 21 aus dem Bronchialraum angesaugt. Die an die Austrittsöffnung 21 anschließende trichterförmige Erweiterung 21a verhindert das Zusetzen der Austrittsöffnung 21 und des Lumens 13 mit zähem Sekret, da infolge der trichterförmigen Erweiterung neben dem Sekret noch ein Luftstrom mit angesaugt wird, so daß das zähe Sekret in das Lumen 23 eingesaugt und damit abgeführt werden kann. Die trichterförmige Erweiterung des distalen Endes des Lumens 13 verhindert das Verstopfen bzw. Zusetzen des distalen Endes des Lumens mit Sekret.

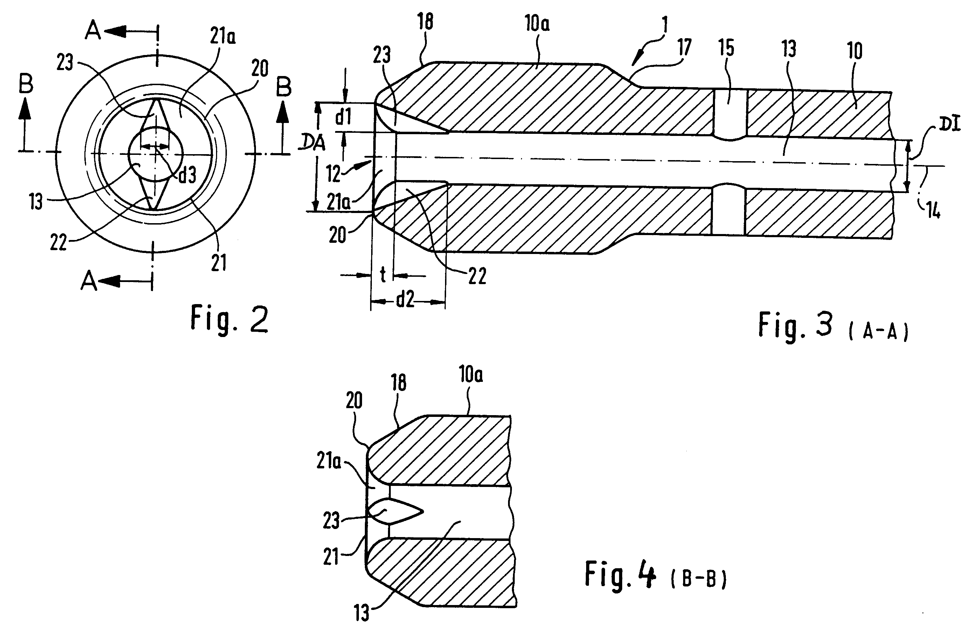

In den Fig. 2 bis 4 ist ein erfindungsgemäßer Absaugkatheter 1 dargestellt, dessen distaler Endbereich eine kleine trichterförmige Erweiterung 21a sowie zusätzliche in die die trichterförmige Erweiterung 21a und das Lumen 13 begrenzende Wandung eingedrückte axial verlaufende Kerben 22, 23 aufweist.

Die distale Austrittsöffnung 21 bildet eine Kreisfläche, deren Querschnitt größer als der Querschnitt des Lumens 13 ist. Diese distale Austrittsöffnung 21 ist durch zusätzliche Kerben 22, 23 in Teilberichen radial erweitert. Diese Kerben 22, 23, die gleichmäßig über den Umfang verteilt sind, beispielsweise zwei einander gegenüberliegende Kerben, wie dargestellt, oder zusätzlich zwei weitere hierzu um 90° versetzte Kerben, vertiefen die trichterförmige Erweiterung 21a an zwei Stellen in radialer Richtung. Diese Kerben 22, 23 verlaufen jedoch nicht nur im Bereich der trichterförmigen Erweiterung 21a, sondern erstrecken sich weiter über den Katheter in axialer Richtung bis hinein in das Lumen 13. Dies ist auch aus der Ansicht nach Fig. 2 ersichtlich.

Die im Bereich des distalen Austrittes des Lumens 13 ausgebildeten Kerben sollten eine Tiefe d1, gemessen von der Lumenwandung in radialer Richtung von d1, von etwa 0,1 bis 0,3 mm aufweisen und eine Länge d2, gemessen vom distalen Ende in axialer Richtung, von etwa 2 bis 4 mm. Die Breite d3 der Kerben, siehe Fig. 2, sollte in jedem Fall kleiner als der Durchmesser des Lumens 13 sein, insbesondere etwa 1/7 bis 1/4 des Durchmessers DI des Lumens 13 betragen. Der Innendurchmesser DI des Lumens 13 beträgt für Kathetergrößen 8 bis 18 CH zwischen 1,0 bis 4 mm.

Die Tiefe t der trichterförmigen Erweiterung sollte zwischen 2 bis 4 mm betragen, der Durchmesser DA der Austrittsöffnung am Ausgang, d.h. im Bereich des Endwulstes 20 etwa 1,25 bis 4,3 mm betragen.

Mit dem erfindungsgemäß ausgebildeten Absaugkatheter, d.h. der Ausbildung des distalen Endbereiches, werden jegliche Verletzungen beim Absaugen der Schleimhäute und auch ein Verstopfen des Absaugkatheters mit Sekreten vermieden.

## Patentansprüche

1. Absaugkatheter (1) zum Absaugen aus dem Trachealbronchialsystem eines Patienten, enthaltend ein biegsames Katheterrohr (10) mit einem proximalen Ende (11) und einem distalen Ende (12) und mindestens einem durchgehend ausgebildeten Lumen (13), wobei das Katheterrohr (10) in einem distalen Endbereich Belüftungsöffnungen (15) aufweist und das Lumen (13) des Katheterrohres am distalen Ende eine trichterartige Erweiterung (21a) bildet, **dadurch gekennzeichnet**, daß in der die trichterartige Erweiterung (21a) begrenzenden Wandung des Katheterrohres (10) mindestens eine sich axial erstreckende Kerbe (22, 23) vorhanden ist.

2. Absaugkatheter nach Anspruch 1,
**dadurch gekennzeichnet**, daß die trichterartige Erweiterung (21a) von dem distalen Ende (12) des Katheters sich bis zu einer Position des Lumens (13) erstreckt, die etwa 2 mm bis 4 mm von dem distalen Ende (12) des Katheterrohres entfernt ist.

3. Absaugkatheter nach Anspruch 1,
**dadurch gekennzeichnet**, daß in der trichterartigen Erweiterung (21a) mindestens zwei gleichmäßig verteilt angeordnete Kerben (22, 23) ausgebildet sind.

4. Absaugkatheter nach Anspruch 3,
**dadurch gekennzeichnet**, daß die gleichmäßig verteilt angeordneten Kerben (22, 23) so ausgebildet sind, daß sie axial entlang des Lumens (13) sich gleich weit mit der trichterartigen Erweiterung (21a) erstrecken.

## Claims

1. Suction catheter (1) for sucking from the tracheobronchial system of a patient, comprising a flexible catheter tube (10) with a proximal end (11) and a distal end (12) and at least one through lumen (13), said catheter tube (10) having ventilation openings (15) in a distal end area, and the lumen (13) of the catheter tube (10) form a funnel-shaped expansion (21a) at the distal end characterized in that at least one axially extending notch (22, 23) is provided in the wall of the catheter tube (10) that delimits the funnel-shaped expansion (21a).

2. Suction catheter according to claim 1 characterized in that funnel-shaped expansion (21a) extends from the distal end (12) of the catheter up to a position of lumen (13) that is approximately 2 mm to 4 mm away from distal end (12) of the catheter tube.

3. Suction catheter according to claim 1 characterized in that at least two uniformly distributed notches (22, 23) are formed in funnel-shaped expansion (21a).

4. Suction catheter according to claim 3 characterized in that uniformly distributed notches (22, 23) are so formed that they extend axially along lumen (13) for the same distance as funnel-shaped expansion (21a).

## Revendications

1. Cathéter d'aspiration (1) servant à l'aspiration du système trachéobronchique d'un patient, comprenant un tube cathéter flexible (10) avec une extrémité proximale (11) et une extrémité distale (12) et au moins un percement (13) continu, le tube cathéter (10) présentant des orifces d'aération (15) ménagés dans une zone d'extrémité distale, et le percement (13) du tube cathéter formant, à l'extrémité distale, un évasement (21a) en forme d'entonnoir, caractérisé en ce qu'au moins une entaille (22, 23) d'orientation axiale est prévue dons la paroi du tube cathéter (10) délimitant l'évasement (21a) en forme d'entonnoir.

2. Cathéter d'aspiration selon la revendication 1, caractérisé en ce que l'évasement (21a) en forme d'entonnoir s'étend de l'extrémité distale (12) du cathéter jusqu'à un endroit du percement (13) qui est distant de 2 mm à 4 mm, environ, de l'extrémité distale (12) du tube cathéter.

3. Cathéter d'aspiration selon la revendication 1, caractérisé en ce qu'au moins deux entailles (22, 23) réparties régulièrement sont prévues dans l'évasement (21 a) en forme d'entonnoir.

4. Cathéter d'aspiration selon la revendication 3, caractérisé en ce que les entailles (22, 23) réparties régulièrement sont réalisées de façon à ce qu'elles s'étendent axialement le long du percement (13) et sur la même distance que l'évasement (21a) en forme d'entonnoir.
